# EUROPÄISCHE PATENTSCHRIFT

(11) **EP 0 560 133 B1**
(45) Veröffentlichungstag und Bekanntmachung des Hinweises auf die Patenterteilung: **20.01.1999**
(21) Anmeldenummer: 93102923.5
(22) Anmeldetag: 25.02.1993
(51) Int. Cl.: B65B 55/10

(54) **Verfahren zum Sterilisieren von Hohlkörpern und Vorrichtung zur Durchführung des Verfahrens**
Method for sterilising hollow bodies and device for executing the method
Procédé pour stériliser des corps creux et dispositif pour la mise en oeuvre du procédé

(30) Priorität: 12.03.1992 DE 4207896
(43) Veröffentlichungstag der Anmeldung: 15.09.1993
(73) Patentinhaber: Tetra Laval Holdings & Finance SA, 1009 Pully (CH)
(72) Erfinder: Reil, Wilhelm, CH-6140 Bensheim (CH); Liebram, Udo, CH-6102 Pfungstadt (CH); Vögele, Peter, Dr., W-6100 Darmstadt (DE)
(74) Vertreter: Weber, Dieter, Dr.

(56) Entgegenhaltungen:
- EP-A- 0 453 942
- DE-A- 3 220 451

## Beschreibung

Die Erfindung betrifft ein Verfahren zum gleichzeitigen Sterilisieren von wenigstens zwei Hohlkörpern, insbesondere Verpackungen für fließfähige Lebensmittel, bei weichem die Hohlkörpergleichzeitig in einen länglich ausgestalteten, geschlossenen Schleusenraum gebracht, in diesem eine Zeit lang einem gasförmigen Sterilisierungsmedium ausgesetzt und nach dem Sterilisieren aus dem Schleusenraum herausgeführt werden, die Hohlkörper in dem Schleusenraum aus einer Einlaßposition um eine Drehachse intermittierend über mindestens zwei Zwischenpositionen zu einer Ausführposition gedreht werden, wobei Ruhezeiten in den jeweiligen Positionen liegen, und in wenigstens einer Zwischenposition das Sterilisiermedium in das Innere des Hohlkörpers eingeführt und von dort außerhalb abgenommen wird und in wenigstens einer dieser Zwischenposition nachgeschalteten Zwischenposition das Sterilisiermedium aus den Hohlkörpern entfernt wird.

Für die Verpackung von Lebensmitteln, z.B. das aseptische Verpacken von Milch, sind Sterilisierverfahren bekannt. Bei einigen werden die als Hohlkörper bezeichneten Verpackungen durch ein Feld energiereicher Strahlen geführt, die von Elektronenkanonen erzeugt werden, so daß nahezu alle Bakterien in der Verpackung bzw. auf ihrer Oberfläche abgetötet werden. Bei wieder anderen Sterilisierungsverfahren werden sterilisierende Mittel aufgestäubt, um dadurch die Packungsoberflächen aseptisch zu machen.

Zumeist bestand die Schwierigkeit, den üblichen Außenraum, der bakteriell stark belastet ist, gegen einen aseptischen Innenraum einer Maschine abzudichten und gleichwohl zu sterilisierende Hohlkörper durch die dichtende Fläche hindurchzuführen. Dies geschah mit Hilfe von Schleusen. Von dieser Art Vorrichtungen gibt es verschiedene bekannte Ausführungen, wie z.B. die Drehtürausführung, bei welcher die Packungen die dichtende Fläche in ähnlicher Weise durchtreten wie der Kunde die Außentür eines Warenhauses von der kalten Umgebung in das geheizte Innere und umgekehrt. Bei diesen Drehtürschleusen konnte im allgemeinen immer nur ein Hohlkörper nach dem anderen eingeführt und sterilisiert werden. Außerdem wurde der jeweilige Hohlkörper den sterilisierenden Strahlen oder Flüssigkeiten in einem einzigen Raum, vorzugsweise in einer einzigen Ruheposition, ausgesetzt und verließ danach den Schleusenraum, um in den aseptischen Innenraum der Maschine einzutreten. Bisweilen war die Sterilisierung mit Nachteil nicht vollständig gelungen. Als besonders nachteilig hat es sich gezeigt, daß bei der Verwendung von flüssigen sterilisierenden Mitteln das aseptische Füllgut mit Restflüssigkeitsmengen vermischt wurde, weil es bei den bekannten Schleusen innerhalb des einzigen Raumes nicht gelingen konnte, das sterilisierende Mittel von den Oberflächen des Hohlkörpers vollständig zu entfernen.

Aus der EP-A-0.453.942 ist ein Sterilisierverfahren ähnlich der eingangs genannten Art bekannt. Dort befinden sich auf einem Rotor in einer Station zwei im Umfang des Rotors nebeneinander angeordnete, voneinander getrennte Schleusenräume, in welche über Kanäle Sterilisiermedium gleichzeitig nach innerhalb und außerhalb des Zuschnittes einer Packung eingeführt wird, parallel vertikal nach oben wegströmt, gesammelt und über einen Auslaßkanal abgeführt wird. Bei dieser Art Sterilisierung werden große Mengen an Sterilisiermittel verbraucht. Abgesehen von der gleichzeitigen Sterilisierung von jeweils immer nur zwei unfertigen Zuschnittenund der damit verbundenen geringen Leistung der Gesamtmaschine ist das vollständige Entfernen von sterilisierendem Mittel unzureichend, denn von der Zuführposition der Verpackungszuschnitte zu deren Abführposition gibt es nur zwei Zwischenpositionen.

Nach einem weiterhin aus der DE-A-32 20 451 bekannten Verfahren werden verunreinigte Flaschen auf einer Transportvorrichtung hintereinander stehend einzeln in Flaschenaufnahmezellen befördert, wobei die jeweils vorderste Flasche gegen einen vertikalen Riemenförderer abkippt und von diesem hochgeschoben wird. In jeder Flaschenaufnahmezelle befindet sich eine Flasche ohne Hinweise auf größere Flaschenaufnahmräume.

Bei der bekannten Vorrichtung muß jede Flaschenaufnahmezelle unten und oben offen sein und hat also Gaskontakt mit allen anderen Zellen und auch mit den Flaschen. Der Fachmann liest aus der Veröffentlichung allenfalls, daß mit der bekannten Vorrichtung Flaschen vielleicht keimfrei gemacht, sicher aber nicht sterilisiert werden, denn die offene Flasche steht nahezu auf ihrem gesamten Weg durch die Maschine mit keimhaltiger Luft in Verbindung.

Das Entkeimen mittels eines Tauchbades, das Austropfenlassen der gefüllten Flaschen, das Einsprühen von sterilem Wasser durch eine Düse unterhalb des Flaschenhalses erlaubt bis auf das kurzzeitige Einführen der Einblasdüse nicht eine durchgreifende Entkeimungs- oder gar Sterilisierungsbehandlung, zumal der erwähnte Gaskontakt bei dem weiteren Transport mit keimhaltigem Gas diese Behandlung wieder zunichte machen würde.

Der Erfindung liegt die Aufgabe zugrunde, ein Sterilisierungsverfahren der eingangs genannten Art zuverlässiger und leistungsstärker auszugestalten und nach dem Aufbringen des sterilisierenden Mittels nach vollständiger Sterilisierung dieses Mittel vom Hohlkörpervollständig zu entfernen, ohne daß der Verbrauch an sterilisierendem Mittel zu groß wird und dennoch eine durchgreifende und intensive Sterilisierung der Hohlkörper gewährleistet ist. Dabei soll besonders auf die Schleusenfunktion des Sterilisierverfahrens geachtet und diese vorteilhaft ausgebaut werden. Ferner soll eine Vorrichtung geschaffen werden, die nach einem Verfahren der vorstehenden Art betrieben werden kann.

Diese Aufgabe wird für das Verfahren erfindungsgemäß dadurch gelöst, daß sich die Drehachse des Schleusenraumes horizontal erstreckt und daß eine Reihe von mehr als zwei, in einem Strang an einem Stück fest aneinanderhaftenden Hohlkörpern, die an ihrem der Drehachse zugeordneten Ende offen sind, in Richtung auf die Drehachse zu radial in den Schleusenraum eingeführt wird, wobei in dem Schleusenraum befindliche Einströmrohre in die Hohlkörper eindringen, so daß das Sterilisiermedium nur in das innere des Hohlkörpers eingeführt und danach vom Grund des Schleusenraumes, vorbei an der gesamten Außenwandung des Hohlkörpers, radial nach außen abgezogen wird.

Die Leistung der Hohlkörper bearbeitenden und verarbeitenden Maschinen kann bei gleichzeitigem Einführen mehrerer, z. B. fünf oder mehr Hohlkörper entsprechend vervielfältigt werden. Es wird gemäß der Erfindung bewußt ein Strang von Hohlkörpern von z.B. fünf, zehn, fünfzehn oder mehr Hohlkörpern in einer Reihe hintereinander gleichzeitig radial in einen Schleusenraum eingeführt, der wie eine Aufnahmebehäler arbeitet und sich selbst entsprechend der Reihe von Hohlkörpern längs erstreckt, wobei diese Erstreckung in der Horizontalen liegt. Die Leistung der Maschine ist gegenüber dem bekannten Verfahren durch die gleichzeitige Behandlung der Vielzahl von Hohlkörpern erhöht. Damit die Positionierung, Behandlung und Bearbeitung der Hohlkörper exakt erfolgt, befinden sich diese an einem Stück und haften fest aneinander, so daß der Abstand von einem Hohlkörper zum nächsten exakt definiert ist und die Füllung und Entleerung der Hohlkörper bei gleichzeitiger Behandlung mit großer Stückzahl pro Zeiteinheit, d.h. mit großer Leistung erfolgen kann. Gleichwohl leidet die intensive und durchgreifende Sterilisierung dadurch in keiner Weise.

Diese Leistungssteigerung verbessert sich auch noch dadurch, daß die Drehung der Hohlkörper in den sternartig angeordneten Schleusenräumen um eine horizontale Drehachse erfolgt. Hier kann man an geeigneten Stellen die Schwerkraft zu Hilfe nehmen und Bewegungen z.B. beim Ein- und Ausladen des Stranges von Hohlkörpern beschleunigen und sicherer ausführen. Das durchgreifende Sterilisieren ergibt sich besonders günstig und intensiv, wenn das Sterilisiermedium zuerst nur in das Innere des Hohlkörpers eingeführt wird, an der Innenwand unter Sterilisierung dieser Oberfläche entlangstreicht, dann die Richtung umkehren muß, über die äußere Oberfläche zurückläuft und erst dann von außerhalb abgenommen wird. In zuverlässiger Weise gelangen sämtliche Oberflächen des Hohlkörpers mit dem Sterilisiermedium in Kontakt, und das gleichzeitig bei einer Vielzahl von Hohlkörpern. Das erfindungsgemäße Verfahren erlaubt eine unerwartete Steigerung der Kapazität bei gleichzeitiger Verlängerung der Bearbeitungsphasen.

Ferner sind mehrere Positionen vorgesehen, in welchen die zu sterilisierenden Hohlkörper Ruhezeiten haben, so daß auf engem Raum ausreichend Zeit für die Sterilisierung und Trocknung der Hohlkörper bleibt. Während selbstverständlich eine Einlaßposition und eine Ausführposition vorhanden sein müssen, sind erfindungsgemäß dazwischen mindestens zwei Zwischenpositionen vorgesehen. Falls es tatsächlich nur zwei Zwischenpositionen sind, kann in der einen, in Bewegungsrichtung vordersten Zwischenposition das Sterilisiermedium an einer Stelle zugeführt und an einer anderen Stelle abgeführt werden, vorzugsweise wenn der Hohlkörper sich in dieser Zwischenposition in Ruhe befindet. In der wenigstens einen nachgeschalteten Zwischenposition verbleibt dann die gleiche Zeit wie in der ersten Position zum Entfernen des Sterilisiermediums aus dem Hohlkörper, so daß die oben beschriebenen Nachteile bekannter Verfahren entfallen.

Weiterhin ist es vorteilhaft, wenn erfindungsgemäß die Luft in dem Sterilisiermedium als Träger für das sterilisierende Mittel zu ihrer Entkeimung gefiltert wird. Im folgenden wird zwischen einem sterilisierenden Mittel und dem Sterilisiermedium unterschieden, wobei in entsprechend konditionierte Luft das sterilisierende Mittel - vorzugsweise durch Zerstäuben einer Flüssigkeit - so eingeführt wird, daß sich dadurch das Sterilisiermedium bildet, welches die physikalischen Eigenschaften eines Gases hat und sich daher besonders für das erfindungsgemäße Sterilisierverfahren eignet. Die Entkeimung von Luft durch Wärme und Filter ist bekannt, und es sind auch verschiedene sterilisierende Mittel bekannt, von denen eines beispielsweise Wasserstoffperoxid ist (H₂O₂). Dieses Mittel läßt sich beispielsweise gut in entkeimte Luft eindüsen, und dann liegt ein gut pumpfähiges und durch Leitungen führbares Sterilisiermedium vor.

Vorteilhaft ist es gemäß der Erfindung weiterhin, wenn das Sterilisiermedium nach dem Sterilisieren des Hohlkörpers in wenigstens einer Zwischenposition durch Einleiten steriler Luft und Abziehen von außen von dem Hohlkörper entfernt wird, wenn der Kreislauf für sterile Luft wenigstens im Bereich des Schleusenraumes vom Kreislauf für Sterilisiermedium getrennt ist und wenn das Sterilisiermedium durch Einbringen eines sterilisierenden Mittels in derart kleinem Abstand vor dem Eintritt in den Hohlkörper in sterile Luft erzeugt wird, daß das Sterilisiermedium außreichend konzentriert ist. Man kann also einen größeren Teil eines Kreislaufes als einen solchen für sterile Luft ausgestalten und bringt erfindungsgemäß das sterilisierende Mittel erst direkt vor dem Eintritt in den Hohlkörper in diese sterile Luft ein, so daß dadurch das Sterilisiermedium erzeugt wird und dort zur Verfügung steht, wo es benötigt wird, nämlich im Hohlkörper zu dessen Sterilisierung.

Das Applizieren von Sterilisiermedium auf die äußeren und inneren Oberflächen eines Hohlkörpers gelingt auf diese Weise auch bei mehreren Hohlkörpern gleichzeitig, vorzugsweise von innen nach außen. Gleichzeitig ist erfindungsgemäß dafür gesorgt, daß aus dem ohnehin vorhandenen Kreislauf für sterile Luft solche in einer anderen, nachgeschalteten Zwischenposition eingeleitet wird, um das Entfernen etwa am Hohlkörper noch haftenden Sterilisiermediums zu besorgen.

Besonders vorteilhaft ist es dabei, wenn man die sterile Luft erwärmt, wobei hier vorzugsweise an 130°C gedacht ist, weil dann ein optimaler Trocknungseffekt der sterilen Luft zum Entfernen des verdampfungsfähigen Sterilisiermediums möglich ist.

Es ist einerseits von einem Kreislauf für sterile Luft und andererseits von einem Kreislauf für Sterilisiermedium gesprochen, also zwei wenigstens teilweise voneinander getrennten Kreisläufen, die aber einfach geführt werden können, weil die Möglichkeit besteht, überwiegend den Kreislauf für sterile Luft vorzusehen. Durch diese Kreisläufe kann erfindungsgemäß sterilisierendes Mittel und überhaupt Energie (z.B. Wärmeenergie) eingespart werden, weil insbesondere das sterilisierende Mittel wiederverwendbar im Kreislauf umgewälzt wird. Auch bei teilweise offenen Kreisläufen tut das Ansaugen von Frischluft dem keinen Abbruch.

Mit dem erfindungsgemäßen Verfahren kann man das Sterilisiermedium mit den zu sterilisierenden Oberflächen des Hohlkörpers in Verbindung bringen, indem man das Sterilisiermedium sich dort kondensieren läßt oder auch alternativ, indem man es an den Oberflächen in höherer Konzentration vorbeistreichen läßt. Gewünschtenfalls können die Kreisläufe vollständig geschlossen gehalten werden, wenn dies günstige Einsparmöglichkeiten an sterilisierendem Mittel und Energie bedeutet.

Bei der Bewegung des zu sterilisierenden Hohlkörpers in der beschriebenen intermittierenden Weise ist es möglich, zahlreiche Zwischenpositionen zwischen der Einlaßöffnung und der Ausführöffnung der Trommel anzuordnen, nach dem Einbringen von Sterilisiermedium z.B. drei Positionen zum Entfernen des Sterilisiermediums, beispielsweise durch Trocknen. Die Anzahl der Positionen, einschließlich Einlaßposition und Ausführposition beträgt erfindungsgemäß vorzugsweise mindestens vier und höchstens zwölf, so daß außer den Einlaß- und Ausführpositionen zwei bis zehn Zwischenpositionen zur Verfügung stehen. Ersichtlich ergeben sich hierdurch vorteilhaft variable Möglichkeiten, unter Ausgestaltung des Sterilisierungsverfahrens als Schleuse mit verhältnismäßig großer Leistung Hohlkörper innen und außen zuverlässig zu sterilisieren und ausreichend Zwischenpositionen zum Nachbehandeln, z.B. Entfernen von Sterilisiermedium, vorzusehen.

Die Erfindung betrifft ferner eine Vorrichtung zum Sterilisieren von wenigstens zwei Hohlkörpern, insbesondere Verpackungen für Lebensmittel, mit einer Trommel mit Einlaßöffnung, einem Schleusenraum und einem den Hohlkörper durch eine Sterilisierungsposition und weitere Behandlungspositionen im inneren der Trommel bewegenden Förderer.

Bekannte Förderer sind solche mit translatorischer Bewegung, wo zu sterilisierende Hohlkörper, z.B. Fließmittelpackungen, von einem ersten Förderband in der bakteriell belasteten Außenluft durch Anheben in einen Schleusenraum geschoben, nach dem einmaligen Anhalten herausgeführt und im aseptischen Innenraum der Maschine auf einem weiteren Förderer zu nachfolgenden Behandlungsstationen weitergeleitet wird, wobei schließlich der Austritt durch eine Drehtürschleuse erfolgt.

Andere bekannte Sterilisiervorrichtungen sind außerordentlich aufwendig, insbesondere wenn die Schleusenfunktion ausgebildet ist.

Es besteht daher die ähnliche Aufgabe wie oben in Verbindung mit dem Verfahren erwähnt, wobei hier Vorrichtungsmerkmale zu finden sind, um ein Verfahren ähnlich dem vorstehend beschriebenen durchführen zu können.

Erfindungsgemäß wird für die Vorrichtung die Aufgabe dadurch gelöst, daß der Schleusenraum in der stationären, stirnseitig durch Scheiben geschlossenen Trommel mit zylindermantelförmigen Trommelwandungen gebildet ist, der Förderer als um eine zentrisch in der Trommel gelagerte Drehachse intermittierend angetriebener Stern mit einer Nabe und N radial von dieser nach außen ragenden, im Querschnitt U-förmigen Schleusenräumen ausgebildet ist, wobei N = 4 bis 12 ist, daß in der Trommelwandung in einer Einlaßposition eine Einlaßöffnung, daneben in einer Ausführposition eine Ausführöffnung und in weiteren Winkelabständen dazu in Zwischenpositionen Anschlüsse für Leitungen und daß im Stern für jeden Schleusenraum getrennte Zuführleitungen mit daran anschließenden, in die Schleusenräume ragenden Einströmrohren angeordnet sind. Der Förderer wird erfindungsgemäß also als Rotor ausgestaltet, wobei ein Stern intermittierend um eine Drehachse rotiert, die mit der Trommelachse zusammenfällt. Der Stern besteht aus einer Nabe, von deren äußerer Oberfläche in der Anzahl N U-förmige Schleusenräume so angebracht sind, daß das U nach außen offen ist, so daß der zu sterilisierende Hohlkörper radial von außen in den jeweiligen, wie einen Aufnahmebehälter arbeitenden Schleusenraum eingeschoben werden kann. Das Innere der Trommel umgibt den rotationsfähigen Stern und kann doch in einfacher Weise stationär zuverlässig aufgebaut sein. Die äußere Oberfläche der Trommel ist grob gesehen ein Zylinder. Vorne und hinten befinden sich stationäre Scheiben an den Stirnseiten, und diese Scheiben sind mit den zylindermantelförmigen Trommelwandungen verbunden. Es ist einfache und beherrschbare Technik, abgedichtet durch die stirnseitigen Scheiben eine Welle für den rotationsfähigen Stern vorzusehen. Der Innenraum der Trommel enthält mindestens einen Schleusenraum, der vorübergehend beim jeweiligen Beladen mit bakteriell beladener Außenluft in Berührung kommt, und zwar über die Einlaßöffnung an der einen Stelle der Trommelwandung. Ansonsten ist das gesamte Innere der Trommel aseptisch, denn in den in Winkelabständen angeordneten Behandlungspositionen befindet sich eingeführtes Sterilisiermedium, und die Ausführöffnung an der anderen Stelle im Umfang der Trommelwandung ist - vorzugsweise über einen aseptischen Schleusenvorraum - mit dem aseptischen Innenraum der Maschine verbunden. Damit ist eine Vorrichtung zum Sterilisieren in Schleusengestalt geschaffen, mit der bei kompakter Bauweise auf engem Raum eine zuverlässige und günstige Sterilisierung möglich ist, welche die oben erwähnten Nachteile entbehrt. Zwischen der Einlaßöffnung und der Ausführöffnung ist wie bei einer Pufferstrecke eine Kreisbahn mit einer entsprechenden Anzahl N von Behandlungspositionen vorgesehen, so daß viel Trocknungszeit oder Zeit zum Entfernen von Sterilisiermedium verbleibt, auch wenn der Takt der intermittierenden Bewegung des Sternes drei Sekunden oder auch vier Sekunden beträgt; ein Takt, der für eine hochleistungsfähige Packungsmaschine in der Technik heute angestrebt wird. Als Hohlkörper kann man wie beim Stand der Technik Verpackungen nehmen.

Die neue Vorrichtung gemäß der Erfindung kann aber auch besonders vielseitig ausgestaltet werden, denn sie hat zahlreiche getrennte Anschlüsse für die Zufuhr und Abfuhr von Medien. In Verbindung mit der vorliegenden Erfindung werden Luft und sterilisierendes Mittel und - sich daraus ergebend - Sterilisiermedium erwähnt und beschrieben. Es ist aber ebenso denkbar, daß man die an den stationären Anschlüssen anbringbaren Leitungen auch mit anderen Medien beschickt, so daß man die Vorrichtung zum Sterilisieren gleichzeitig auch für andere Funktionen einsetzen kann. Beispielsweise könnten zwischen der Einlaßöffnung und der Ausführöffnung in weiteren Zwischenpositionen außer dem Sterilisieren auch andere Behandlungen zusätzlich noch nachgeschaltet werden, so daß man hier sehr variable Prozesse gestalten kann

Die Vorrichtung ist erfindungsgemäß besonders günstig weiter dann ausgestaltet, wenn das an der Zuführleitung angeschlossene Einströmrohr sich etwa mittig im Schleusenraum radial nach außen zu dessen offenem Ende hin über etwa 1/4, vorzugsweise 2/3, der Höhe des Schleusenraumes erstreckt, und wenn die Wände des Schleusenraumes an seinem äußeren, offenen Ende eine Dichtlippe aufweisen. Hier ist der oben schon erwähnte Aufbau des Sternes genauer beschrieben mit der zusätzlichen Maßnahme, daß sich in jedem Schleusenraum etwa mittig mindestens ein Einströmrohr befindet, das sich in der beschriebenen Länge radial nach außen erstreckt, wodurch die Möglichkeit vorgegeben ist, Sterilisiermedium zuerst durch dieses Einströmrohr in den Hohlkörper einzuführen, so daß dessen Innenflächen zuverlässig und schnell sterilisiert werden. In Verbindung mit der stationären Trommelwandung bildet jeder Schleusenraum mit Hilfe seiner Dichtlippen am offenen Ende des U einen mehr oder weniger gasdichten Raum, in welchem sich eine aseptische Atmosphäre aufbauen kann.

Zweckmäßig ist der zu sterilisierende Hohlkörper in Verbindung mit dem Einströmrohr so ausgestaltet, daß er an seinem der Trommelmitte zugeordneten Ende offen ist. Dadurch kann der Hohlkörper beim Einführen durch die Einlaßöffnung über und um das Einströmrohr herumgeschoben werden, so daß sich dessen offenes freies Ende letztlich im Bereich des Bodens des Hohlkörpers befindet. Der Fachmann erkennt, wie zuverlässig die Sterilisierung mit dieser erfindungsgemäßen Vorrichtung durchführbar ist, denn das frische Sterilisiermedium gelangt zunächst in den Innenraum des Hohlkörpers im Bereich seines Bodens; dort, wo bei den bekannten Sterilisiergeräten häufig die schwächste Sterilisierung erfolgt.

Günstig ist es gemäß der Erfindung weiterhin, wenn die Nabe und die U-förmigen Schleusenräume des Sterns axial derart langgestreckt ausgebildet sind, daß mehrere Hohlkörper hintereinander von den U-förmigen Schleusenräumen aufnehmbar und intermittierend drehbar sind, daß mehrere Einströmrohre entsprechend der Anzahl der aufnehmbaren Hohlkörper axial hintereinander in dem jeweiligen Schleusenraum angebracht und mit jeweils einem Hauptzuführungsrohr in der Nabe verbunden sind und daß jeder feste Anschluß in der Trommelwandung mit einer parallel zum jeweiligen Hauptzuführungsrohr verlaufenden Sammlerbrücke verbunden ist. Die Leistung der Sterilisiervorrichtung kann durch diese Maßnahme ganz erheblich gesteigert werden. Selbstverständlich können nicht nur zwei Hohlkörper hintereinander, sondern es können fünf, zehn, fünfzehn oder mehr Hohlkörper in einer Reihe hintereinander gleichzeitig von einem Schiebestempel durch die Einlaßöffnung über das Einströmrohr in den Schleusenraum eingeschoben werden, um danach im Laufe der Rotationsbewegung die beschriebenen Behandlungen zu erfahren. Nach dem Einschieben der Reihe der mehreren Hohlkörper durch die Einlaßöffnung dichtet der Schiebestempel die Trommel im Bereich der Einlaßöffnung ab, obwohl die Wandungen des Schleusenraumes die Reihe von Hohlkörpern von diesem Schiebestempel auf die innere Oberfläche der Trommelwandung herunterschieben kann, nämlich in die einzelnen Zwischenpositionen. Während sich die jeweilige Hohlkörperreihe ruhend in den Zwischenpositionen befindet, kann der Schiebestempel wieder unter Freilegung der Einlaßöffnung zurückgezogen, mit neuen Hohlkörpern beschickt werden und den nächsten, leeren Schleusenraum füllen.

Besonders zweckmäßig ist die Anwendung einer langgestreckt ausgestalteten Sterilisiervorrichtung gemäß der Erfindung bei der Sterilisierung von durch Tiefziehen, Spritzen oder Zusammenschweißen hergestellten Kunststoffpackungen, obgleich auch andere Hohlkörper ebenso vorteilhaft verarbeitet werden können. Es sind bereits Herstellungsverfahren für Hohlkörper vorgeschlagen worden, bei welchen fünf bis zehn Hohlkörper an einem Stück hängend wie ein Strang von der Maschine gefertigt und abgegeben werden, so daß es außerordentlich günstig ist, eine Sterilisiervorrichtung der hier beschriebenen Art mit Schleusenfunktion zu haben, weil dann solche Packungen auch zum Verpacken aseptischer Güter, z.B. Lebensmittel, eingesetzt werden können. Der Strang von Hohlkörpern ist also eine Reihe von fest aneinanderhaftenden, bis auf die Oberseite im Bereich einer Öffnung geschlossenen Körper, die mit ihrem geschlossenen Boden beispielsweise auf den beschriebenen Schiebestempel aufgeschoben und von diesem durch die Einlaßöffnung in die Trommel und in den Schleusenraum hineingebracht werden können.

In jeder rotationsfähigen Nabe befinden sich soviele Hauptzuführungsrohre, die sich über die gesamte axiale Länge der Nabe erstrecken, wie Schleusenräume außen an der Nahe sternförmig abstehen. Diese Hauptzuführungsrohre können als Sackbohrungen ausgeführt sein, wobei sie am einen, dem sogenannten vorderen Ende offen und am gegenüberliegenden Ende geschlossen sind. Dem offenen Ende gegenüber befindet sich die stationäre Scheibe, die vorzugsweise gleich viele feste Anschlüsse hat, und zwischen der stillstehenden Scheibe und der rotierenden Nabe befinden sich Dichtungsstellen, um das Entweichen etwa zugeführter Gase oder Flüssigkeiten auszuschalten. An der stirnseitigen stationären Scheibe sind also entsprechend viele Anschlüsse vorhanden, die alle einzeln oder auch gemeinsam an Leitungen von einem oder mehreren Kreisläufen angeschlossen werden können. Alternativ und vorzugsweise ist es möglich, zwischen einigen der Hauptzuführungsrohre Querverbindungsleitungen vorzusehen, so daß von einer äußeren Leitung ein bestimmtes Medium, z.B. sterile Luft, gleichzeitig zu verschiedenen Hauptzuführungsrohren gebracht werden kann. Das gleiche ist allerdings auch ohne Leitungen in der Nabe möglich, wenn die Zuführleitung außen mit mehreren der erwähnten Anschlüsse für die Hauptzuführungsrohre verbunden ist. Diese Maßnahme ist wichtig für die beschriebene bevorzugte Ausführungsform, bei welcher sterile Trocknungsluft gleichzeitig in vier Hauptzuführungsrohre in die Nabe und damit in vier Schleusenräume eingeführt wird. Es versteht sich, daß von jedem Hauptzuführungsrohr entsprechend der Anzahl einzuführender Hohlkörper mehrere Einströmrohre angebracht sind, weil jeder einzelne Hohlkörper in der gewünschten zuverlässigen Weise durch die erfindungsgemäße Vorrichtung sterilisiert werden soll.

Auch abzugsseitig kann die langgestreckte Ausbildung des rotationsfähigen erfindungsgemäßen Sterns gleichermaßen günstig und gewünschtenfalls multifunktional ausgestaltet werden. Jedem Hauptzuführungsrohr, welches sich radial innenseitig befindet, entspricht eine außen parallel zu diesem Hauptzuführungsrohr verlaufende Sammlerbrücke. Dies ist ein Rohr, welches verschiedene Öffnungen in der Trommelwandung außen verbindet und zu einem Anschluß vereint, falls von allen Hohlkörpern eines Stranges dasselbe Medium in einen Kreislauf abgeführt werden soll. Die Einlaß- und Auslaßöffnungen sind bei dieser langgestreckten Ausbildung ebenfalls wie langgestreckte Schlitze ausgestaltet.

Die Erfindung ist vorteilhaft weiter dadurch ausgestaltet, daß der feste Anschluß wenigstens einer ersten Zwischenposition und das diesem festen Anschluß jeweils gegenüberliegende Hauptzuführungsrohr in der Nabe mit Leitungen eines Kreislaufes für Sterilisiermedium verbunden sind, während die anderen Anschlüsse und Hauptzuführungsrohre mit Leitungen eines Kreislaufes für sterile Luft verbunden sind. Auch die Vorrichtung arbeitet nach dem Prinzip zweier wenigstens streckenweise getrennt gehaltener Kreisläufe, nämlich des einen Kreislaufes für das Sterilisiermedium und des anderen Kreislaufes für sterile Luft. Die Ausgestaltung in der vorstehend beschriebenen Weise sieht für den Kreislauf für Sterilisiermedium nur eine Zwischenposition vor, während die andere - oder wenn es mehrere andere gibt - die anderen Zwischenpositionen alle dem Kreislauf für sterile Luft zugeordnet werden. Diese Ausgestaltung der Vorrichtung hat den Vorteil, daß in einer Position sterilisiert werden kann und in der nachfolgenden oder sogar in mehreren Schritten nachfolgend in mehreren Zwischenpositionen Sterilisiermedium zuverlässig und vollständig aus dem Hohlkörper entfernt werden kann.

Erfindungsgemäß ist es weiterhin von Vorteil, wenn in dem Kreislauf für sterile Luft eine Katalysator, ein Vorfilter, ein Gebläse, ein Absolutfilter und eine Heizung liegen; und wenn in dem Kreislauf für Sterilisiermedium ein Gebläse, ein Absolutfilter, eine Heizung und eine Düse für die Zugabe von sterilisierendem Mittel liegen. Der Katalysator sorgt für die Vernichtung des in der sterilen Luft noch vorhandenen sterilisierenden Mittels, wobei z.B. H₂O₂ auf diese Weise gut aus dem Fließmittel entfernbar ist. Dieses Herausziehen von sterilisierendem Mittel mit Hilfe des Katalysators ist für jeden Kreislauf für sterile Luft erforderlich, weil dieser Kreislauf vor dem neuen Einsatz in der Trommel im wesentlichen frei von sterilisierendem Mittel sein soll. Dadurch ergeben sich gute Voraussetzungen für Abziehen von sterilisierendem Mittel in Trocknungspositionen oder dergleichen. In einem Vorfilter kann zusätzliche Frischluft angesaugt werden, um die sterile Luft entsprechend zu konditionieren. Das Gebläse sorgt für die Umwälzung, der Absolutfilter hilft beim Entkeimen der Luft, und die Heizung ist besonders günstig, wenn die sterile Luft zum Trocknen verwendet werden soll.

Auch der Kreislauf für Sterilisiermedium erfordert das Gebläse für die Umwälzung und den Absolutfilter zur Entkeimung, während die Düse, welche im Zuströmbereich der Trommel vorgesehen ist, das sterilisierende Mittel in der richtigen Menge - vorzugsweise durch ein Regelgerät gesteuert - zuführt. Die Heizung dient wiederum der geeigneten Konditionierung.

Verwendet der Fachmann die erfindungsgemäß gelehrten Einheiten zur Bildung der vorgesehenen Kreisläufe, dann sind verschiedene Schaltungen möglich, die nachfolgend in Verbindung mit drei unterschiedlichen und bevorzugten Ausführungsformen beschrieben werden.

Bei einer bevorzugten Ausführungsform ist es für die erfindungsgemäße Vorrichtung besonders zweckmäßig, wenn zwischen einer Vereinigungsstelle, an welcher beide Kreisläufe zusammenlaufen, und einer Gabelungsstelle, an welcher sich der eine Kreislauf vom anderen trennt, eine gemeinsame Leitung mit Katalysator, Vorfilter, Gebläse und Absolutfilter für beide Kreisläufe geführt ist. Durch einen solchen Aufbau kann man in der gemeinsam geführten Leitung zahlreiche Einheiten einsparen, denn über eine Strecke zwischen der erwähnten Vereinigungsstelle und der Gabelungsstelle können beide Kreisläufe durch dieselben Einheiten geführt werden. Entsprechend der Aufgabenstellung kann diese vereinfachte Anlage besonders preiswert sein und dennoch eine zuverlässige Sterilisierung mit Schleusenfunktion ermöglichen.

Weitere Vorteile, Merkmale und Anwendungsmöglichkeiten der vorliegenden Erfindung ergeben sich aus der folgenden Beschreibung bevorzugter Ausführungsbeispiele in Verbindung mit den Zeichnungen. Es zeigen:
- Figur 1: eine vertikale Querschnittsansicht durch die Sterilisiervorrichtung mit den in der Trommel befindlichen Schleusenräumen, wobei Einrichtungen für die Zuführung und für das Herausschieben der Hohlkörper schematisch dargestellt sind,
- Figur 2: eine zur Schnittebene der Figur 1 senkrechte und ebenfalls vertikale Schnittansicht der Vorrichtung entlang der Linie II-II der Figur 1,
- Figur 3: eine erste Ausführungsform der an die Sterilisiervorrichtung angeschlossenen Kreisläufe,
- Figur 4: eine zweite Ausführungsform der an die Sterilisiervorrichtung angeschlossenen Kreisläufe und
- Figur 5: eine dritte andere Ausführungsform von an die Sterilisiervorrichtung angeschlossenen Kreisläufen.

Auf dem Boden 1 ist über ein nur schematisch dargestelltes Stützgestell 2 eine Trommel 3 gehaltert, welche die Form eines Zylinders hat und hohl ist. Der Hohlraum im Inneren 4 der Trommel 3 ist von den zylindermantelförmigen Trommelwandungen 5, der ebenen kreisrunden hinteren Scheibe 6 und der vorderen Scheibe 7 umschlossen. Die Trommel 3 mit ihren Trommelwandungen 5 und stirnseitigen Schreiben 6 und 7 ist stationär, während sich in ihrem Inneren 4 ein allgemein mit 8 bezeichneter Stern um eine mittige horizontale Drehachse 9 drehen kann, die mit der Achse der Trommel 3 zusammenfällt. Auf einer in den stationären Scheiben 6 und 7 gelagerten Welle 10 ist dieser Stern 8 über Lagerböcke 11 drehbar angetrieben und besteht aus einer länglichen, etwa im Querschnitt sechseckigen Nabe 12, von welcher radial sechs ebenfalls längliche, im Querschnitt U-förmige Schleusenräume 13 so befestigt sind, daß diese Schleusenräume 13 nach außen zur Trommelwandung 5 hin offen und gegenüber der Trommelwandung 5 über Dichtlippen 14 abgedichtet sind. Die innere ringförmige Oberfläche der Trommelwandung 5 ist glatt, so daß sich der Stern 8 in Richtung des Pfeiles 15 um die horizontale Drehachse 9 drehen kann.

Bei der hier dargestellten Ausführungsform besteht das Innere 4 der Trommel 3 aus sechs im Querschnitt etwa segmentförmigen Abschnitten, die mit Luft gefüllt sind. Bei einer anderen, hier nicht dargestellten Ausführungsform kann man zwischen zwei in den erkennbaren Winkelabständen benachbarten Schleusenräumen 13 je eine segmentartige Abtrennwand einbauen. Das Wesen der Funktion der Sterilisiervorrichtung ändert sich dadurch aber nicht.

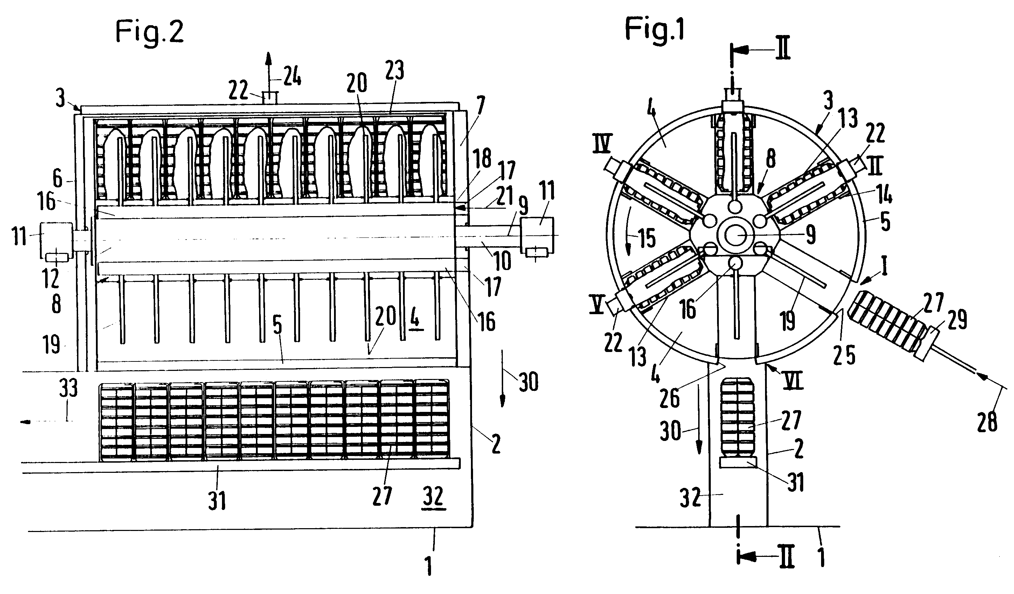

In der Nabe 12 verlaufen hinter jeder Ebene, an welcher der wie ein Aufnahmebehälter arbeitende Schleusenraum 13 befestigt ist, ein Hauptzuführungsrohr 16. Im Falle der hier dargestellten Ausführungsform, bei welcher der Stern 8 sechs radial nach außen von der Nabe 12 abstehende und an der Nabe 12 angebrachte Schleusenräume 13 hat, verlaufen in der Nabe 12 also auch sechs Hauptzuführungsrohre 16, die an ihrem hinteren Ende (in Figur 2 links) geschlossen und an ihrem vorderen Ende offen sind. In der vorderen stationären Scheibe 7 sind ebenso viele Löcher wie Hauptzuführungsrohre 16 in der Nabe 12 sind, und diese Löcher 17 liegen in der jeweiligen Ruheposition der intermittierend gedrehten Nabe 12 in Flucht zu den Hauptzuführungsrohren 16. Selbstverständlich sind im Betrieb und nach fertig montierter Anlage nicht dargestellte Anschlüsse mit den Löchern 17 verbunden, so daß die Löcher 17 mit Leitungen von Kreisläufen verbunden werden können. Zwischen der stationären Scheibe 7 und der vorderen Stirnfläche der Nabe 12 befinden sich Dichtungseinrichtungen, so daß die Hauptzuführungsrohre 16 während der Rotationsbewegung des Sterns 8 nach vorn abgedichtet sind und in der jeweiligen Ruheposition, wie in den Figuren hier gezeigt, Dichtungsstellen 18 dafür sorgen, daß aus Leitungen über die Löcher 17 in der Scheibe 7 in die Hauptzuführungsrohre 16 eintretende Medien nicht zur Seite austreten können.

Stattdessen ist mit jedem Hauptzuführungsrohr 16 eine Reihe von zehn Einströmrohren 19 verbunden, die etwa mittig in dem jeweiligen Schleusenraum 13 ebenfalls von der Nabe 12 radial nach außen über etwa 3/4 der Höhe der U-förmigen Schleusenräume 13 verlaufen und an ihrem freien Ende 20 offen sind. Ein in Richtung des Pfeiles 21 (Figur 2 rechts) in das jeweilige Hauptzuführungsrohr 16 eintretendes Medium kann also durch zehn Einströmrohre 19 und über deren freies offenes Ende 20 in den Schleusenraum 13 einströmen.

An der stationären Trommelwandung 5 befinden sich in Positionen II, III, IV und V Anschlüsse 22, deren jeder über eine Sammlerbrücke 23 verbunden ist. In der zylindermantelförmigen Trommelwandung 5 befindet sich also bei der hier gezeigten Ausführungsform an den vier Positionen II, III, IV und V jeweils eine Sammlerbrücke 23 mit einem Anschluß 22. Die Sammlerbrücke 23 erstreckt sich ebenfalls parallel zu den Hauptzuführungsrohren 16 und parallel zur Drehachse 9 des Sterns 8 derart, daß der gesamte längliche Raum innerhalb des jeweiligen Schleusenraumes 13 abgedeckt und mit dem Anschluß 22 in Verbindung steht. Die aus den offenen Enden 20 der Einströmrohre 19 austretenden Medien können auf diese Weise nämlich leicht in die Sammlerbrücke und über diese zu dem jeweiligen Anschluß 22 gelangen, so daß eine Abnahme dieser Medien gemäß Pfeil 24 in Figur 2 oben möglich ist.

In den Positionen I und VI gibt es keine Sammlerbrücken und Anschlüsse. Stattdessen ist die Trommelwandung 5 hier schlitzartig geöffnet, so daß sich in der Position I eine Einlaßöffnung 25 und in der Position VI eine Ausführöffnung 26 ergeben. Beide Öffnungen 25, 26 sind so lang wie der jeweilige Schleusenraum 13 und so breit, daß die zu sterilisierenden Hohlkörper 27 von außerhalb in den Innenraum der Trommel 3, d.h. den Schleusenraum 13, in Richtung des Pfeiles 28 (radial) von einem Schiebestempel 29 eingeschoben werden können. Jeweils an der ersten Einlaßposition I wird also der Schleusenraum 13 beladen, indem der sich gerade dort befindende Schleusenraum 13 beladen wird, während nach einem Umlauf von etwa 270° in Richtung des Pfeiles 15 (intermittierend) nach der Sterilisierung die sterilisierten Hohlkörper 27 in Richtung des Pfeiles 30 auf einen nachgeschalteten Förderer 31 in einem aseptischen Vorraum 32 ausgeladen werden. Nach dem Einschieben der noch bakteriell verunreinigten Hohlkörper 27 in Richtung Pfeil 28 dichtet der Schiebestempel 29 die Einlaßöffnung 25 ab, und dann beginnt die erste Drehbewegung um 45° in die Position II. Die sterilisierte Reihe von Hohlkörpern 27 wird schließlich in dem aseptischen Vorraum 32 in Richtung des Pfeiles 33 zur weiteren Behandlung abgeführt.

Bei der hier gewählten speziellen Ausführungsform der Schleusenräume 13 mit den mittig angeordneten Einströmrohren 19 wird vorausgesetzt, daß die zu sterilisierenden Hohlkörper 27 an ihrem oberen, dem Schiebestempel 29 gegenüberliegenden Ende ein Loch haben, so daß sich beim Einschieben gemäß Pfeil 28 der Hohlkörper 27 über das Einströmrohr 19 und um dieses herum geschoben wird. In den Positionen II, III, IV und V sind die Hohlkörper 27, die beispielsweise Flüssigkeitsverpackungen sein können, im Schnitt dargestellt, wobei jeweils radial außen der Boden der Hohlkörper 27 und gegenüber das Loch vorzustellen sind, durch welches das Einströmrohr 19 in den Hohlkörper eingedrungen ist und welches später die Anbringung einer Öffnungsvorrichtung ermöglicht. Für die hier gezeigte Ausführungsform ist vorgesehen, daß zehn Hohlkörper an einem Stück aus Kunststoff gebildet und in Gestalt dieser Zehnerreihe in der hier beschriebenen Vorrichtung sterilisiert wird. Deshalb ragen zehn Einströmrohre 19 in die teilweise aufgebrochen in Figur 2 dargestellten Hohlkörper bis fast zu deren Boden. Durch diesen Aufbau in Verbindung mit diesem Typ Hohlkörper wird erreicht, daß ein Sterilisiermedium oder ein anderes Gas nach Austritt aus dem Hauptzuführungsrohr 16 über das Einströmrohr 19 bis fast an den Boden des Inneren des Hohlkörpers 27 gelangt, von dort entlang den Innenwandungen des Hohlkörpers bis zu dessen Loch strömt, dort austritt und dann die Strömungsrichtung umkehrt, um nämlich an der gesamten Außenwandung des zu sterilisierenden oder zu trocknenden Hohlkörpers 27 vorbeizustreichen und schließlich über die Sammlerbrücke 23 und den Anschluß 22 abgeführt zu werden. Die Zufuhr von Strömungsmedien erfolgt bei der hier gezeigten Vorrichtung also vorzugsweise (in Figur 2 von rechts nach links) durch die Löcher 17 in der stationären Scheibe 7, durch die Hauptzuführungsrohre 16, die Einströmrohre 19 in die durch die jeweiligen Aufnahmebehälter 13 gebildeten Teilräume; und die Abführung dieser Fließmedien gelingt durch die Sammlerbrücke 23 und den Anschluß 22 zur Ableitung gemäß Pfeil 24 (Figur 2).

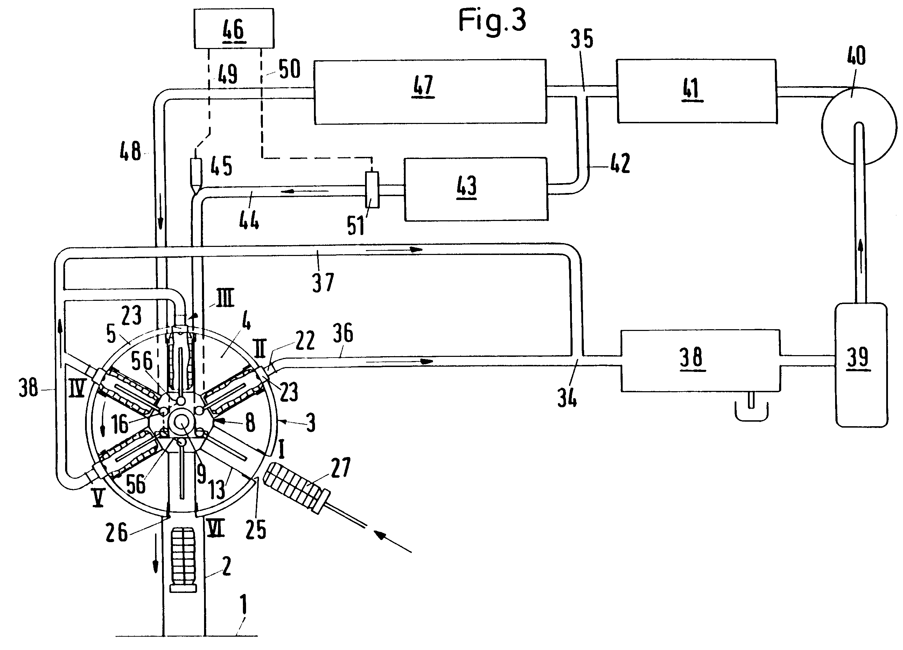

Den Aufbau der vorstehend beschriebenen Schleusentrommel vorausgesetzt, wird nun anhand der Figuren 3 bis 5 die Führung verschiedener Kreisläufe für die Aufbereitung von Strömungsmedien beschrieben.

In Figur 3 ist die erste Ausführungsform gezeigt, bei welcher der Kreislauf für Sterilisiermedium ein Stück weit gemeinsame Einrichtungen und Leitungen mit dem Kreislauf für sterile Luft hat. Diese gemeinsame Leitungsführung liegt zwischen der Vereinigungsstelle 34 und einer Gabelungsstelle 35, wobei die in den verschiedenen Leitungen in den Figuren 3 bis 5 gezeigten Pfeile die Strömungsrichtung darstellen. Das durch das Hauptzuführungsrohr 16 in Position II eingeleitete und am Anschluß 22 in Position II verbrauchte Sterilisiermedium strömt durch die Leitung 36 zu der erwähnten Vereinigungsstelle 34, wo es sich nämlich mit verbrauchter - weil teilweise mit Sterilisiermedium angereicherter - steriler Trockenluft vereinigt, die über Leitung 37 aus Leitung 38 von den Anschlüssen 22 in den Positionen III, IV und V kommt. Der nach der Vereinigungsstelle 34 gemeinsame Strom gelangt in den Katalysator 38', wo aus dem Sterilisiermedium das sterilisierende Mittel, bei einem Beispiel H₂O₂, abgezogen bzw. entfernt wird, so daß der weitere Strom im wesentlichen sterile Luft enthält, die dann in den Vorfilter 39 eintritt, wo teilweise Frischluft zusätzlich angesaugt wird. Das nun teilweise bakteriell belastete Gemisch wird durch das erste Gebläse 40 in den nachgeschalteten ersten Absolutfilter 41 gedrückt, von wo es als sterile Luft die Gabelungsstelle 35 erreicht.

Von hier trennen sich die beiden Kreisläufe für sterile Luft einerseits und für Sterilisiermedium andererseits wie folgt.
1. Durch die Leitung 42 gelangt ein Teil der sterilen Luft in eine erste Heizung 43, wo die Luft auf beispielsweise etwa 130°C erhitzt wird, um einen guten Aufnahmeeffekt zu bieten. Von hier strömt die sterile Trocknungsluft durch Leitung 44, wo mittels einer Düse 45 von einem Vorratsbehälter 46 sterilisierendes Mittel eingedüst wird, z.B. mittels einer Ultraschalldüse, so daß direkt vor dem Eingang in das Hauptzuführungsrohr 16 in der ersten Zwischenposition II Sterilisiermedium, in der richtigen Weise konditioniert, vorhanden ist und dem Innenraum des zu sterilisierenden Hohlkörpers 27 zugeführt werden kann. Dies war der Kreislauf für Sterilisiermedium.
2. Der Kreislauf für sterile Luft trennt sich an der Gabelungsstelle 35 von dem aus dem ersten Absolutfilter 41 austretenden Luftgemisch und tritt in eine zweite Heizung 47 ein, in welcher die sterile Luft ebenfalls auf z.B. etwa 130°C erwärmt wird. Von dort strömt die warme sterile Trocknungsluft durch die Leitung 48 in vier der Hauptzuführungsrohre 16, nämlich in die der Positionen III, IV, V und VI. Bei dieser Ausführungsform kann in drei Zwischenpositionen III, IV und V ein zuverlässiges Entfernen etwaigen Sterilisiermediums aus dem Hohlkörper erfolgen, vorzugsweise in drei Stufen zu je drei bis vier Sekunden Dauer ausgetrocknet werden. In der Ausführposition VI verläßt dann die Reihe der Hohlkörper 27 die Trommel 3 dann einwandfrei sterilisiert.

Man kann gemäß der gestrichelten Linien 49 und 50 in Figur 3 auch eine variabel dosierte Zugabe von sterilisierendem Mittel aus dem Vorratsbehälter 46 vorsehen, wobei die Dosierung über einen Sensor 51 erfolgt, welcher die Temperatur, gegebenenfalls Konzentration des Fließmediums an gesuchten Mitteln oder dergleichen abtastet und durch eine elektrische Leitung 50 Informationen an den Vorratsbehälter 46 liefert. Eine einfachere Variante besteht darin, daß aus der in Figur 3 gezeigten Schaltung der Sensor 51 und die Leitung 50 gestrichen sind.

Eine andere Alternative der Figur 3 ist zur Einsparung der Heizung 47 möglich, indem man nämlich die Gabelungsstelle 35 hinter die erste Heizung 43 legt, so daß von dort sowohl die Leitung 48 als auch die Leitung 44 mit steriler Trocknungsluft versorgt werden.

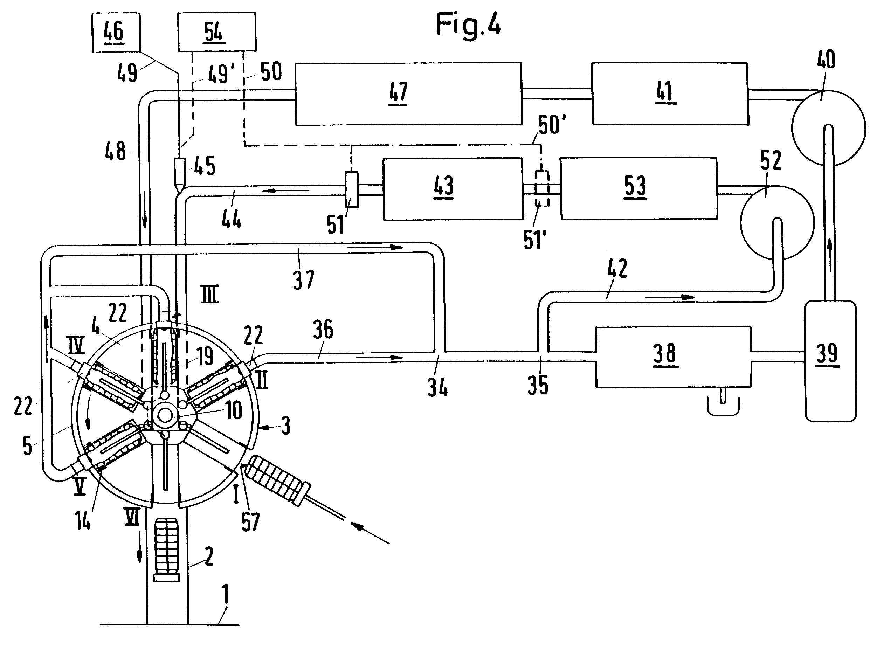

Die zweite andere Ausführungsform ist in Figur 4 gezeigt. Dort gibt es zwar die gleiche Vereinigungsstelle 34, wo aus den Leitungen 36 und 37 Sterilisiermedium und mit solchem beladene Trocknungsluft vereinigt werden, die Gabelungsstelle 35 liegt hier aber bereits vor dem Katalysator 38, so daß nur ein Teil des Gemisches die Einheiten Katalysator 38, Vorfilter 39, erstes Gebläse 40, ersten Absolutfilter 41 und Heizung 47 zur Leitung 48 durchströmt, während ein anderer Teil durch die Leitung 42 von einem zweiten Gebläse 52 durch einen zusätzlichen Absolutfilter 53 und durch die nachgeschaltete erste Heizung 43 gedrückt wird, um wieder in die schon beschriebene Leitung 44 zu gelangen. Der Vorteil dieses Aufbaues gemäß Figur 4 gegenüber dem der Figur 3 liegt darin, daß nicht das gesamte sterilisierende Mittel in dem Katalysator 38 aus dem Gemisch entfernt werden muß, um nachträglich durch die Düse 45 wieder eingegeben zu werden. Hierdurch wird also sterilisierendes Mittel gespart.

Bei dieser Ausführungsform kann gemäß den gestrichelten Linien 49' und 50 eine Steuerung der eingedüsten Menge des sterilisierenden Mittels vorgenommen werden. In der Leitung 44 ist wieder hinter der Heizung 43 ein Sensor 51 eingebaut, welcher über die Leitung 50 ein Signal an den Regler 54 gibt, durch welchen über die elektrische Leitung 49 eine Steuerung der Düse 45 angesteuert wird, um mehr oder weniger sterilisierendes Mittel aus dem Vorratsbehälter 46 zuzulassen.

Eine weitere andere Alternative ist entsprechend der strichpunktierten Leitung 50' möglich, welche nämlich den Regler 54 mit dem an anderer Stelle plazierten Sensor 51' verbindet. Bei dieser Ausführungsform liegt der Sensor 51' im kalten Bereich der Leitung 44, d.h. vor der Heizung 43, wenn man die Strömungsrichtung beachtet. Das Messen der gewünschten Größen, wie z.B. Konzentration an sterilisierendem Mittel, ist auf diese Weise unter Umständen einfacher und zuverlässiger.

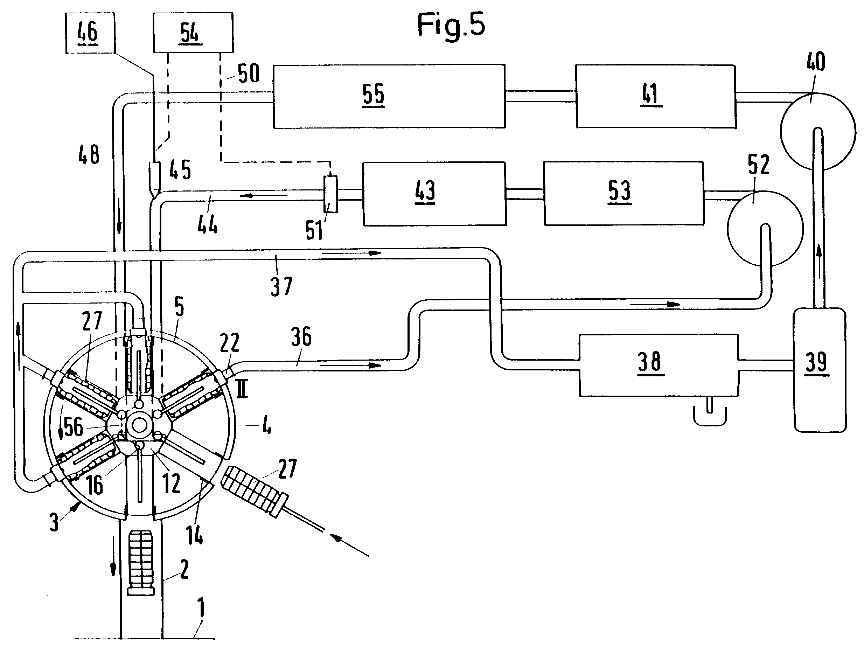

Die letzte und dritte hier gezeigte Ausführungsform wird anhand Figur 5 beschrieben. Hier sind die Kreisläufe a) für sterile Luft einerseits und b) für Sterilisiermedium andererseits vollständig voneinander getrennt. Es gibt hier keine Vereinigungs- und auch keine Gabelungsstelle. Vom Anschluß 22 der Position II der Trommel 3 strömt wieder verbrauchtes Sterilisiermedium durch Leitung 36 zum zweiten Gebläse 52, um von letzterem durch den zusätzlichen Absolutfilter 53 und die erste Heizung 43 in die schon beschriebene Leitung 44 gedrückt zu werden. Der Sensor 51 tastet die gesuchten Größen ab, mißt z.B. die Konzentration an sterilisierendem Mittel und veranlaßt über den Regler 54 eine Zugabe an sterilisierendem Mittel aus dem Vorratsbehälter 46 mittels Düse 45 in das durch die Leitung 44 strömende Sterilisiermedium. Das Eindüsen 45 erfolgt in geringem Abstand direkt vor dem Hauptzuführungsrohr 16 in Position II, so daß diese Position die Sterilisierposition ist. Der Kreislauf für Sterilisiermedium ist damit in sich geschlossen.

Getrennt davon ist der Kreislauf für sterile Luft geschaltet. Wie bei den anderen Ausführungsformen strömt die gesammelte und mit Sterilisiermedium belastete Trocknungsluft durch die Leitung 37 zum Katalysator 38, wie bei der ersten Ausführungsform nach Figur 3. Der weitere Verlauf der Strömung der sterilen Luft in diesem Kreislauf ist ähnlich wie bei Figur 3, nur daß die sterile Luft nach Verlassen des ersten Absolutfilters 41 in eine getrennte Heizung 55 und von dort in die bereits beschriebene Leitung 48 gelangt, ähnlich wie bei dem Kreislauf der Figur 4. Die gestrichelte Linie 56 stellt eine Verbindungsleitung dar zwischen den einzelnen Hauptzuführungsrohren 16 in den Positionen III, IV, V und VI. Wenn sterile Trocknungsluft auch die Ausführposition VI erreicht und dort abströmt, so ist dies keineswegs schädlich, denn dadurch wird der aseptische Vorraum 32 steril gehalten.

Im Betrieb wird bei der Herstellung von Flüssigkeitspackungen eine Reihe von zehn Packungen aus Kunststoff in einem Strang, sozusagen einstückig hergestellt und auf den Schiebestempel 29 verbracht. Dieser bewegt die Packungsreihe 27 in Richtung des Pfeiles 28 in den leeren Aufnahmebehälter 13 in der Einlaßposition I, bis der Schiebestempel 29 die Einlaßöffnung 25 verschließt. Dann beginnt sich der Stern 8 in Pfeilrichtung 15 um 45° zu drehen, so daß die hier betrachtete Packungsreihe 27 in der ersten Zwischenposition II, welche die Sterilisierposition ist, zum Stillstand gebracht wird. Zwischen den einzelnen Positionen gleiten die Dichtlippen 14 über die Trommelwandung 5.

In der Sterilisierposition II gelangt das Sterilisiermedium in der beschriebenen Weise durch das dieser Packungsreihe zugeordnete Hauptzuführungsrohr 16 und über die zehn Einströmrohre 19 in die zehn einzelnen Packungen. Aus deren Öffnung, die in Figur 4 mit 57 bezeichnet ist, strömt das Sterilisiermedium aus dem Inneren der Packung 27 heraus, an deren Oberflächen vom Grund des Aufnahmebehälters radial nach außen zum Bereich der Dichtlippen 14 und innerhalb derselben über die Sammlerbrücke 23 in den Anschluß 22, um von dort durch die Leitung 16 abgezogen zu werden.

Alle drei oder vier Sekunden wird eine Reihe von zehn Packungen erzeugt und in dieser Weise in der Position I in die Trommel 3 eingeladen, so daß zum Durchströmen des Sterilisiermediums in der ersten Zwischenposition II drei bzw. vier Sekunden zur Verfügung stehen. Das Sterilisiermedium kann an den Oberflächen der Packung kondensieren; oder - bei einer anderen Ausführungsform - kann konzentrierteres Sterilisiermedium auch nur an den Packungswänden vorbeistreichen, um dadurch die Sterilisierung zu bewirken.

In jedem Falle ist der Raum in der sterilisierten Packung und in dem betreffenden Aufnahmebehälter 13 mit Sterilisiermedium gefüllt, welches nun ausgetrieben werden muß.

Nach den drei bzw. vier Sekunden Verweilzeit dreht sich der Stern 8 wiederum um 45°, so daß die betrachtete Reihe Kunststoffpackungen 27 nun die obere Position III erreicht, nämlich die erste Trocknungsposition. Aus der Leitung 48 wird das dieser Zwischenposition III zugeordnete Hauptzuführungsrohr 16 mit steriler Trocknungsluft versorgt, die ebenfalls über das Einströmrohr 19 zunächst das Innere der Packung und danach das Äußere trocknet, wonach die mit Sterilisiermedium teilweise belastete Luft nach oben über den Anschluß 22 in der Position III abgezogen und über die beschriebene Leitung 38, 37 abgezogen wird. Auch diese Trocknung erfolgt drei bzw. vier Sekunden lang, wonach der Stern 8 wiederum um 45° in Pfeilrichtung 15 weitergeschaltet wird. Jetzt hat die Packungsreihe die Position IV erreicht, in welcher ebenso wie in der nachfolgenden Zwischenposition V die Trocknung in derselben Weise erfolgt wie vorstehend beschrieben.

Nach der letzten drei bzw. vier Sekunden dauernden Trocknung in der Zwischenposition V gelangt die Packungsreihe dann in die Ausführposition VI, in welcher drei bzw. vier Sekunden zum Abführen der Packungsreihe in Richtung des Pfeiles 30 zur Verfügung stehen.

## Patentansprüche

1. Verfahren zum gleichzeitigen Sterilisieren von wenigstens zwei Hohlkörpern (27), insbesondere Verpackungen für fließfähige Lebensmittel, bei welchem die Hohlkörper (27) gleichzeitig in einen länglich ausgestalteten, geschlossenen Schleusenraum (13) gebracht, in diesem eine Zeit lang einem gasförmigen Sterilisierungsmedium ausgesetzt und nach dem Sterilisieren aus dem Schleusenraum (13) herausgeführt werden, die Hohlkörper (27) in dem Schleusenraum (13) aus einer Einlaßposition (I) um eine Drehachse (9) intermittierend über mindestens zwei Zwischenpositionen (II-V) zu einer Ausführposition (VI) gedreht werden, wobei Ruhezeiten in den jeweiligen Positionen (I-VI) liegen, und in wenigstens einer Zwischenposition (II) das Sterilisiermedium in das Innere des Hohlkörpers (27) eingeführt und von dort außerhalb abgenommen wird und in wenigstens einer dieser Zwischenposition (II) nachgeschalteten Zwischenposition (III-V) das Sterilisiermedium aus den Hohlkörpern (27) entfernt wird, **dadurch gekennzeichnet**, **daß** sich die Drehachse (9) des Schleusenraumes (13) horizontal erstreckt und daß eine Reihe von mehr als zwei, in einem Strang an einem Stück fest aneinanderhaftenden Hohlkörpern (27), die an ihrem der Drehachse (9) zugeordneten Ende offen sind, in Richtung auf die Drehachse (9) zu radial in den Schleusenraum (13) eingeführt wird, wobei in dem Schleusenraum (13) befindliche Einströmrohre (19) in die Hohlkörper (27) eindringen, so daß das Sterilisiermedium nur in das Innere des Hohlkörpers (27) eingeführt und danach vom Grund des Schleusenraumes (13), vorbei an der gesamten Außenwandung des Hohlkörpers (27), radial nach außen abgezogen wird.

2. Verfahren nach Anspruch 1, dadurch gekennzeichnet, daß Luft in dem Sterilisiermedium als Träger für das sterilisierende Mittel zu ihrer Entkeimung gefiltert (41, 53) wird.

3. Verfahren nach Anspruch 1 oder 2, dadurch gekennzeichnet, daß das Sterilisiermedium nach dem Sterilisieren des Hohlkörpers (27) in wenigstens einer Zwischenposition (III-V) durch Einleiten steriler Luft und Abziehen von außen von dem Hohlkörper (27) entfernt wird, daß der Kreislauf für sterile Luft (37, 38 - 41, 48) Wenigstens im Bereich des Schleusenraumes (13) vom Kreislauf für Sterilisiermedium (36, 42 - 45) getrennt ist und daß das Sterilisiermedium durch Einbringen eines sterilisierenden Mittels in derart kleinem Abstand vor dem Eintritt in den Hohlkörper (27) in sterile Luft erzeugt wird, daß das Sterilisiermittel ausreichend konzentriert ist.

4. Vorrichtung zum Sterilisieren von wenigstens zwei Hohlkörpern (27), insbesondere Verpackungen für Lebensmittel, mit einer Trommel (3) mit Einlaßöffnung (25), einem Schleusenraum (13) und einem den Hohlkörper (27) durch eine Sterilisierposition (II) und weitere Behandlungspositionen (III-V) im Inneren (4) der Trommel (3) bewegenden Förderer (8), dadurch gekennzeichnet, daß der Schleusenraum (13) in der stationären, stirnseitig durch Scheiben (6, 7) geschlossene Trommeln (3) mit zylindermantelförmigen Trommelwandungen (5) gebildet ist, der Förderer (8) als um eine zentrisch in der Trommel (3) gelagerte Drehachse (9) intermittierend angetriebener Stern (8) mit einer Nabe (12) und N radial von dieser nach außen ragenden, im Querschnitt U-förmigen Schleusenräumen (13) ausgebildet ist, wobei N = 4 bis 12 ist, daß in der Trommelwandung (5) in einer Einlaßposition (I) eine Einlaßöffnung (25), daneben in einer Ausführposition (VI) eine Ausführöffnung (26) und in weiteren Winkelabständen dazu in Zwischenpositionen (II-V) Anschlüsse (22) für Leitungen (36, 37, 38) und daß im Stern (8) für jeden Schleusenraum (13) getrennte Zuführleitungen (16) mit daran anschließenden, in die Schleusenräume (13) ragenden Einströmrohren (19) angeordnet sind.

5. Vorrichtung nach Anspruch 4, dadurch gekennzeichnet, daß das Einströmrohr (19) sich etwa mittig im Schleusenraum (13) radial nach außen zu dessen offenem Ende hin über etwa 1/4, vorzugsweise 2/3 der Höhe des Schleusenraumes (13) erstreckt, und daß die Wände des Schleusenraumes (13) an seinem äußeren, offenen Ende eine Dichtlippe (14) aufweisen.

6. Vorrichtung nach Anspruch 4 oder 5, dadurch gekennzeichnet, daß die Nabe (12) und die U-förmigen Schleusenräume (13) des Sterns (8) axial derart langgestreckt ausgebildet sind, daß mehrere Hohlkörper (27) hintereinander von den U-förmigen Schleusenräumen (13) aufnehmbar und intermittierend drehbar sind, daß mehrere Einströmrohre (19) entsprechend der Anzahl der aufnehmbaren Hohlkörper (27) axial hintereinander in dem jeweiligen Schleusenraum (13) angebracht und mit jeweils einem Hauptzuführungsrohr (16) in der Nabe (12) verbunden sind und daß jeder feste Anschluß (22) in der Trommelwandung (5) mit einer parallel zum jeweiligen Hauptzuführungsrohr (16) verlaufenden Sammlerbrücke (23) verbunden ist.

7. Vorrichtung nach einem der Ansprüche 4 bis 6, dadurch gekennzeichnet, daß der feste Anschluß (22) wenigstens einer ersten Zwischenposition (II) und das diesem festen Anschluß (22) jeweils gegenüberliegende Hauptzuführungsrohr (16) in der Nabe (12) mit Leitungen (36) eines Kreislaufes für Sterilisiermedium (36, 42 - 45) verbunden sind, während die anderen Anschlüsse (22) und Hauptzuführungsrohre (16) mit Leitungen (37, 38) eines Kreislaufes für sterile Luft (38 -41, 48) verbunden sind.

8. Vorrichtung nach einem der Ansprüche 4 bis 7, dadurch gekennzeichnet, daß in dem Kreislauf für sterile Luft (38 - 41, 48) ein Katalysator (38), ein Vorfilter (39), ein Gebläse (40), ein Absolutfilter (41) und eine Heizung (43) liegen und daß in dem Kreislauf für Sterilisiermedium (36, 42 - 45) ein Gebläse (40; 52), ein Absolutfilter (41; 53) eine Heizung (43) und eine Düse (45) für die Zugabe von sterilisierendem Mittel liegen.

9. Vorrichtung nach einem der Ansprüche 4 bis 8, dadurch gekennzeichnet, daß zwischen einer Vereinigungsstelle (34), an welcher beide Kreisläufe (38 - 41, 48; 36, 42 - 45) zusammenlaufen, und einer Gabelungsstelle (35), an welcher sich der eine Kreislauf vom anderen trennt, eine gemeinsame Leitung mit Katalysator (38), Vorfilter (39), Gebläse (40) und Absolutfilter (41) für beide Kreisläufe geführt sind.

## Claims

1. A process for the simultaneous sterilisation of at least two hollow bodies (27), in particular packs for foodstuffs which are capable of flow, in which the hollow bodies (27) are simultaneously introduced into a closed lock chamber (13) of an elongate configuration, exposed therein for a time to a gaseous sterilisation medium and after the sterilisation operation moved out of the lock chamber (13), the hollow bodies (27) are intermittently rotated in the lock chamber (13) from an inlet position (I) about an axis of rotation (9) by way of at least two intermediate positions (II-V) to a discharge position (VI), wherein there are rest times in the respective positions (I-VI) and in at least one intermediate position (II) the sterilisation medium is introduced into the interior of the hollow body (27) and from there taken off outside and in at least one intermediate position (III-V) disposed downstream of said intermediate position (II) the sterilisation medium is removed from the hollow bodies (27), characterised in that the axis of rotation (9) of the lock chamber (13) extends horizontally and that a row of more than two hollow bodies (27) which adhere firmly to each other in a line in one piece and which are open at their end associated with the axis of rotation (9) is introduced radially into the lock chamber (13) in a direction towards the axis of rotation (9), wherein flow intake tubes (19) disposed in the lock chamber (13) penetrate into the hollow bodies (27) so that the sterilisation medium is introduced only into the interior of the hollow body (27) and thereafter is drawn off radially outwardly from the bottom of the lock chamber (13), past the entire outside wall of the hollow body (27).

2. A process according to claim 1 characterised in that air in the sterilisation medium as a carrier for the sterilising agent is filtered for removing bacteria therefrom (41, 53).

3. A process according to claim 1 or claim 2 characterised in that the sterilisation medium is removed from the hollow body (27) after sterilisation of the hollow body (27) in at least one intermediate position (III-V) by the introduction of sterile air and the removal thereof from outside, that the circuit for sterile air (37, 38-41, 48) is separate from the circulation for sterilisation medium (36, 42-45) at least in the region of the lock chamber (13), and that the sterilisation medium is produced by introducing a sterilising agent into sterile air at such a small spacing upstream of the entry into the hollow body (27) that the sterilising agent is adequately concentrated.

4. Apparatus for sterilising at least two hollow bodies (27), in particular packs for foodstuffs, having a drum (3) with an inlet opening (25), a lock chamber (13) and a conveyor (8) for moving the hollow body (27) through a sterilisation position (II) and further treatment positions (III-V) in the interior (4) of the drum (3), characterised in that the lock chamber (13) is formed in the stationary drum (3) which is closed at its ends by discs (6, 7) and which has drum walls (5) in the form of a cylindrical casing, the conveyor (8) is in the form of a star (8) which is intermittently driven about an axis of rotation (9) mounted concentrically in the drum (3), the star having a hub (12) and N lock chambers (13) which are of U-shaped cross-section and which project radially outwardly from the hub, wherein N = 4 to 12, that provided in the drum wall (5) in an inlet position (I) is an inlet opening (25), therebeside in a discharge position (VI) a discharge opening (26) and at further angular spacings in relation thereto in intermediate positions (II-V) connections (22) for conduits (36, 37, 38), and that feed conduits (16) which are separate for each lock chamber (13) and which have flow intake tubes (19) adjoining same and projecting into the lock chambers (13) are arranged in the star (8).

5. Apparatus according to claim 4 characterised in that the flow intake tube (19) extends substantially centrally in the lock chamber (13) radially outwardly towards the open end thereof over about one-quarter and preferably two-thirds of the height of the lock chamber (13) and that the walls of the lock chamber (13) have a sealing lip (14) at the outer open end of the lock chamber.

6. Apparatus according to claim 4 or claim 5 characterised in that the hub (12) and the U-shaped lock chambers (13) of the star (8) are of an axially elongate configuration in such a way that a plurality of hollow bodies (27) can be received in succession by the U-shaped lock chambers (13) and are intermittently rotatable, that a plurality of flow intake tubes (19) corresponding to the number of receivable hollow bodies (27) are disposed axially in succession in the respective lock chamber (13) and are each connected to a respective main feed tube (16) in the hub (12) and that each fixed connection (22) in the drum wall (5) is connected to a collecting bridge (23) extending in parallel relationship with the respective main feed tube (16).

7. Apparatus according to one of claims 4 to 6 characterised in that the fixed connection (22) of at least one first intermediate position (II) and the main feed tube (16) in the hub (12), which is respectively disposed in opposite relationship to said fixed connection (22), are connected to conduits (36) of a circuit for sterilisation medium (36, 42-45) while the other connections (22) and main feed tubes (16) are connected to conduits (37, 38) of a circuit for sterile air (38-41, 48).

8. Apparatus according to one of claims 4 to 7 characterised in that disposed in the circuit for sterile air (38-41, 48) are a catalyst (38), a pre-filter (39), a blower (40), an absolute filter (41) and a heating means (43) and that disposed in the circuit for sterilisation medium (36, 42-45) are a blower (40; 52), an absolute filter (41; 53), a heating means (43) and a nozzle (45) for the addition of sterilising agent.

9. Apparatus according to one of claims 4 to 8 characterised in that passed between a junction location (34) at which both circuits (38-41, 48; 36, 42-45) come together and a forking location (35) at which the one circuit separates from the other is a common conduit with catalyst (38), pre-filter (39), blower (40) and absolute filter (41) for both circuits.

## Revendications

1. Procédé pour stériliser simultanément au moins deux corps creux (27), en particulier des emballages pour produits alimentaires liquides, dans lequel les corps creux (27) sont disposés simultanément dans une chambre formant sas (13), fermée, de forme oblongue, y sont exposés pendant un certain temps à un fluide de stérilisation sous forme gazeuse, et, après la stérilisation, sont évacués de la chambre formant sas (13); les corps creux (27) subissent, dans la chambre formant sas (13), et à partir d'une position d'entrée (I), une rotation intermittente, autour de l'axe de rotation (9), et en passant par au moins deux positions intermédiaires (II-V), jusqu'à une position de sortie (VI), des temps de repos étant prévus sur chacune des positions (I-VI); et, sur au moins une position intermédiaire (II), le fluide de stérilisation est introduit dans le volume intérieur du corps creux (27), puis en est évacué vers l'extérieur; et, sur au moins une position intermédiaire (III-V) disposée en aval de cette position intermédiaire (II), on évacue le fluide de stérilisation des corps creux (27), caractérisé en ce que l'axe de rotation (9) de la chambre formant sas (13) a une extension horizontale, et que l'on introduit radialement dans la chambre à sas (13), dans la direction allant vers l'axe de rotation (9), une série d'au moins deux corps creux (27), qui adhèrent fermement les uns aux autres en ligne pour former un bloc unique, et qui sont ouverts au niveau de leur extrémité dirigée vers l'axe de rotation (9), auquel cas des tubes d'injection (19), se trouvant dans la chambre formant sas (13), pénètrent dans les corps creux (27) de façon que le fluide de stérilisation ne soit introduit que dans le volume intérieur du corps creux (27), puis soit évacué radialement vers l'extérieur à partir du fond de la chambre formant sas (13), en passant devant la totalité de la paroi extérieure du corps creux (27)

2. Procédé selon la revendication 1, caractérisé en ce que l'air se trouvant dans le fluide de stérilisation, pour servir de support à l'agent stérilisant, est filtré (41, 53) pour être stérilisé.

3. Procédé selon la revendication 1 ou 2, caractérisé en ce que le fluide de stérilisation, après stérilisation du corps creux (27), est sur au moins une position intermédiaire(III-V) éloigné du corps creux (27) par introduction d'air stérile et aspiration de l'extérieur; que le circuit de l'air stérile (37, 38 - 41, 48) est, au moins dans la zone de la chambre à sas (13), séparé du circuit du fluide de stérilisation (36, 42 - 45); et que le fluide de stérilisation est produit par introduction dans de l'air stérile d'un agent stérilisant, à une distance en avant du point de pénétration dans le corps creux (27) suffisamment petite pour que l'agent stérilisant soit suffisamment concentré.

4. Appareil pour stériliser au moins deux corps creux (27), en particulier des emballages pour produits alimentaires, comportant un tambour (3) muni d'un orifice d'entrée (25), d'une chambre à sas (13) et d'un transporteur (8), qui déplace le corps creux (27) en le faisant passer par une position de stérilisation (II) et par d'autres positions de traitement (III-V) à l'intérieur (4) du tambour (3), caractérisé en ce que la chambre à sas (13) est formée dans le tambour (3), fixe, fermé côté frontal par des disques (6, 7) et comportant des parois latérales cylindriques (5); le transporteur (8) est configuré comme une étoile (8), à entraînement intermittent autour d'un axe de rotation (9) disposé en position centrale dans le tambour (3), l'étoile comportant un moyeu (12) et N chambres formant sas (13), ayant en section transversale la forme d'un U et dépassant vers l'extérieur à partir de cette étoile, où N = 4 à 12; que, dans la paroi (5) du tambour, un orifice d'entrée (25) est disposé sur une position d'entrée (I), et de plus un orifice de sortie (26) est disposé sur une position de sortie (VI), et, sur des positions intermédiaires (II-V), ayant d'autres positions angulaires, des raccords (22) sont prévus pour des conduites (36, 37, 38); et que, dans l'étoile (8), des conduites d'amenée (16), distinctes pour chaque chambre formant sas (13), sont disposées avec des tubes d'injection (19), qui s'y rattachent et qui pénètrent dans les chambres formant sas (13).

5. Appareil selon la revendication 4, caractérisé en ce que le tube d'injection (19), qui s'étend approximativement en position centrale dans la chambre formant sas (13), radialement vers l'extérieur jusqu'à son extrémité ouverte, sur environ 1/4 et de préférence les 2/3 de la hauteur de la chambre formant sas (13), et que les parois de la chambre formant sas (13) présentent une lèvre d'étanchéité (14) au niveau de son extrémité extérieure ouverte.

6. Appareil selon la revendication 4 ou 5, caractérisé en ce que le moyeu (12) et les chambres formant sas (13) en forme de U de l'étoile (8) présente dans la direction axiale une extension longitudinale telle que plusieurs corps creux (27) peuvent être reçus les uns après les autres dans les chambres formant sas (13) en forme de U, et peuvent subir une rotation intermittente ; que plusieurs tubes d'injection (19), en fonction du nombre des corps creux (27) pouvant être mis en place, sont rapportés axialement les uns derrière les autres dans la chambre formant sas (13) correspondante et sont assemblés chacun à un tube principal d'amenée (16) dans le moyeu (12); et que chaque raccord fixe (22) se trouvant dans la paroi(5) du tambour est relié à un pont collecteur (23), qui court parallèlement au tube principal d'amenée (16) correspondant.

7. Appareil selon l'une des revendications 4 à 6, caractérisé en ce que le raccord fixe (22) est assemblé à au moins une première position intermédiaire (II), et le tube principal d'amenée (16), qui dans tous les cas est opposé à ce raccord fixe (22), est dans le moyeu (12) assemblé à des conduites (36) d'un circuit destiné au fluide de stérilisation (36, 42 - 45), tandis que les autres raccords (22) et tubes principaux d'amenée (16) sont assemblés à des conduites (37, 38) d'un circuit destiné à l'air stérile (38 - 41, 48).

8. Appareil selon l'une des revendications 4 à 7, caractérisé en ce qu'on a disposé dans le circuit destiné à l'air stérile (38 - 41, 48) un catalyseur (38), un préfiltre (39), une soufflante (40), un filtre absolu (41) et un chauffage (43), et que l'on trouve dans le circuit destiné au fluide de stérilisation (36, 42 - 45) une soufflante (40 ; 52), un filtre absolu (41 ; 53), un chauffage (43) et une buse (45) pour introduire l'agent stérilisant.

9. Appareil selon l'une des revendications 4 à 8, caractérisé en ce que, entre un point de réunion (34) où convergent les deux circuits (38 - 41, 48 ; 36, 42 - 45) et un point de bifurcation (35) où un circuit se sépare de l'autre, on a une conduite commune, avec catalyseur (38), préfiltre (39), soufflante (40) et filtre absolu (41), pour les deux circuits.
